# EUROPEAN PATENT APPLICATION

(11) **EP 3 085 318 A1**
(43) Date of publication of application: **26.10.2016**
(21) Application number: 15164780.7
(22) Date of filing: 23.04.2015
(51) Int. Cl.: A61B 17/435, H01H 21/32

(54) **MULTI-FUNCTION FOOT SWITCH**

(71) Applicant: Shivani Scientific Industries Private Limited, 401104 Mumbai (IN)
(72) Inventor: Kale, Ravikant, 401104 Mumbai (IN); Modi, Ashish, 401104 Mumbai (IN)
(74) Representative: Nordmeyer, Philipp Werner

(57) **Abstract**

Disclosed is a foot switch to be used in medical equipment for controlling various medical operations. The foot switch may comprise a foot pedal, a pressure sensor, a tilt sensor and a control unit. The pressure sensor may be adapted to sense a pressure applied on the foot pedal. The tilt sensor may be adapted to sense tilting of the foot pedal. The control unit may be configured to receive a first control signal and a second control signal from the pressure sensor and the tilt sensor respectively. The control unit may further be configured to perform a first function and a second function based upon the receipt of the first control signal and the second control respectively. The first function and the second function may be associated with controlling parameters associated with the medical equipment, thereby facilitating to control the medical operations.

## Description

### TECHNICAL FIELD

The present disclosure described herein, in general, relates to a foot switch to be used in a medical field, and more particularly, to an improved, multi-function electrical foot switch used in medical devices for performing various medical operations.

### BACKGROUND

In a medical field, different mobile medical equipments may be provided to the doctor in order to assist him/her for performing medical operations on patients. Many times, while performing the medical operations, the doctor may have to simultaneously handle a plurality of surgical instruments or medical equipments. Serious problems creeps in when the doctor alone cannot handle number of instruments simultaneously on his/her own. In this situation, the doctor has to depend on an assistant who assists him/her in handling few of the plurality of instruments or controlling certain parameters associated with the instruments. It is essential for the doctor to optimally control the parameters, the failure of which may be dangerous to the patient's health.

For example, in an In-vitro Fertilization (IVF) treatment, a needle is inserted into the patient's body for recovering the oocyte. During the insertion of the needle inside the patient's body, the doctor may have to concentrate on other components of the medical equipment. Thus, while performing the treatment, it is important to keep the medical equipment in a steady state and in handy location. Further, when the needle is inserted into the patient's body, the doctor may perform certain movements in order to control the parameters associated with the medical equipment. Such unwanted movements may cause serious injuries to internal organs of the patient's body. Thus, it may be challenge for the doctor to keep the controls of the medical equipment handy when his/her hands are engaged in performing other functions.

In the present day situation, the controlling of multiple functions is being implemented via multiple switches present on a control panel of the medical instruments. However, it is a challenge for the doctor to memorize and associate a specific function to a specific switch of the multiple switches present on the control panel. Further, since one or more functions have to be performed sequentially, there is a chance of accidently activating wrong switch by the doctor through the control panel. This may further lead to complications in diagnosis of the patient or even may cause serious injuries to the organs of the patient.

### SUMMARY

Before the present devices and mechanisms, are described, it is to be understood that this application is not limited to the particular mechanism, devices, apparatus, and methodologies described, as there can be multiple possible embodiments which are not expressly illustrated in the present disclosures. It is also to be understood that the terminology used in the description is for the purpose of describing the particular versions or embodiments only, and is not intended to limit the scope of the present application.

In one implementation, an electrical foot switch is disclosed. The electrical foot switch (hereinafter referred to as a "foot switch") may comprise a foot pedal, a first sensor, a second sensor and a control unit. The first sensor and the second sensor may be positioned within the foot pedal. The first sensor and the second sensor may be adapted to sense a first parameter and a second parameter, respectively, associated with the foot pedal. In an embodiment, the first parameter and the second parameter may be sensed when the foot pedal is in a first position and a second position respectively. Further, the first parameter and the second parameter may be sensed based upon an operative action performed, by a user, on the foot pedal. The foot pedal may further be electrically coupled with the control unit. The control unit may be configured to receive a first control signal or a second control signal from the first sensor or the second sensor respectively. In an embodiment, the first control signal may be received when the foot pedal is in the first position. Alternatively, the second control signal may be received when the foot pedal is in the second position. The control unit may further be configured to perform a first function or a second function based upon the receipt of the first control signal or the second control signal respectively.

In another implementation, a method of operating an electrical foot switch is disclosed. The method may comprise sensing, via a first sensor and a second sensor a first parameter and a second parameter, respectively, associated with a foot pedal. In an embodiment, the first parameter and the second parameter may be sensed when the foot pedal is in a first position and a second position respectively. Further, the first parameter and the second parameter may be sensed based upon an operative action performed by a user. The method may further comprise receiving, by a control unit, a first control signal or a second control signal from the first sensor or the second sensor respectively. In an embodiment, the first control signal may be received when the foot pedal is in the first position. Alternatively, the second control signal may be received when the foot pedal is in the second position. Further, the method may comprise performing, by the control unit, a first function or a second function based upon the receipt of the first control signal or the second control signal respectively.

### BRIEF DESCRIPTION OF THE DRAWINGS

The detailed description is described with reference to the accompanying figures. In the figures, the left-most digit(s) of a reference number identifies the figure in which the reference number first appears. The same numbers are used throughout the drawings to refer like features and components.
Figure 1 illustrates an electrical foot switch 100 (hereinafter also referred to as "foot switch 100) oriented in a first position (position-1) in accordance with an embodiment of the present disclosure.
Figure 2 illustrates various components of the foot switch 100 oriented in a second position (position-2) in accordance with an embodiment of the present disclosure.
Figure 3 illustrates pressing and tilting of the foot switch 100 in accordance with an embodiment of the present disclosure.
Figure 4 has two Figures, 4A and 4B that illustrate circuitry arrangements for deriving analog and digital output signals based upon the orientation of the foot switch in the position-1 and the position-2, respectively, in accordance with an embodiment of the present disclosure.

### DETAILED DESCRIPTION

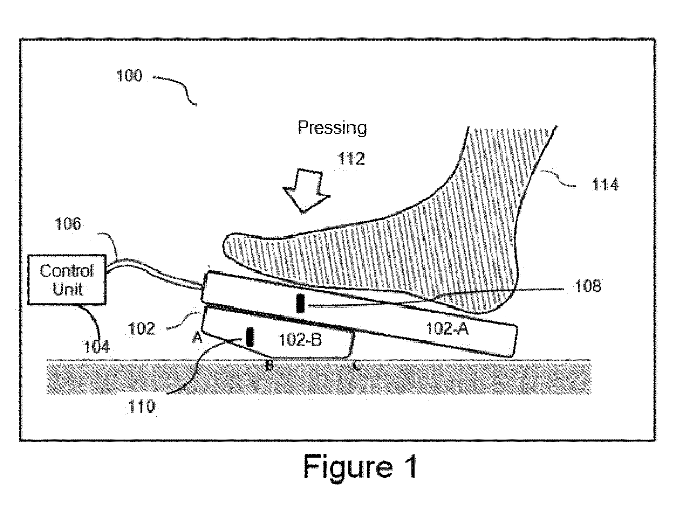

The present disclosure provides an improved foot switch for controlling medical equipment used by a doctor. The foot switch may comprise a foot pedal electrically coupled with a control unit. The foot pedal may comprise a pressure sensor and a tilt sensor. The pressure sensor and the tilt sensor may provide control signals to the control unit for activating and controlling various functions associated with the medical equipment. In an embodiment, the pressure sensor may sense a pressure exerted, by the doctor, on the foot pedal. Further, the tilt sensor may sense change in orientation of the foot pedal, by a predefined angle, based upon tilting of the foot pedal by the doctor. In one embodiment, the control signals provided to the control unit may be based upon the pressure exerted on the foot pedal or the tilting of the foot pedal. Finally, the control unit may be configured to perform a desired medical function/operation based upon the receipt of the control signals. In one embodiment, the control unit may perform a unique function corresponding to a control signal received from each of the pressure sensor and the tilt sensor. In one example, the control unit may control generation of desired level of vacuum for aspirating oocyte from the patient's body. Alternatively, the control unit may remove the blockage in the needle path while aspirating the oocyte from the patient's body. A detail working of the foot switch 100 is further explained, referring to Figures 1, 2, 3 and 4, as below.

Referring to Figure 1, a foot switch 100 integrated with various components is shown in accordance with one embodiment of the present disclosure. As illustrated, the foot switch 100 may comprise a foot pedal 102 and a control unit 104 electrically coupled with the foot pedal 102, via a wire connector 106. The foot pedal 102 comprises a plate 102-A rested upon a block surface 102-B. The foot pedal 102 further comprises a first sensor 108 and a second sensor 110. The first sensor 108 and the second sensor 110 may be positioned within the foot pedal 102. In one embodiment, as shown in Figure 1, the first sensor 108 may be positioned at a location, in the foot pedal 102, wherein the pressure applied upon the foot pedal 102 may be sensed by the first sensor 108. Alternatively, the second sensor 110, as shown in Figure 1, may be positioned at a center of the foot pedal 102 such that the second sensor 110 may sense tilting of the foot pedal 102. In an embodiment, the first sensor 108 may be a pressure sensor selected from a group consisting of a strain-gauge sensor, a capacitive sensor, an electromagnetic sensor, a piezoelectric sensor, an optical sensor and a potentiometric sensor. Further, the second sensor 110 may be a tilt sensor.

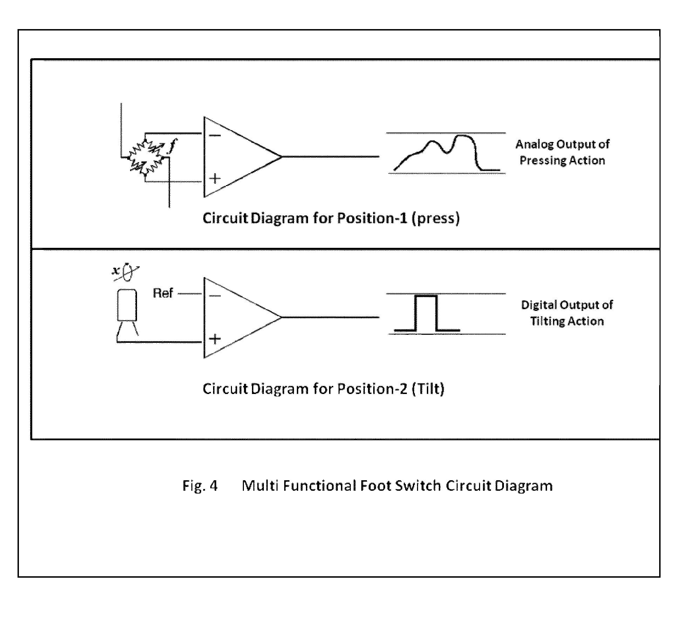

Still referring to figure 1, the foot pedal 102 is shown to be oriented in a first position (position-1). In one embodiment, in the postion-1, the plate 102-A along with the block surface 102-B of the foot pedal 102 may be positioned as shown in figure 1. As illustrated in figure 1, in the postion-1, face "BC" of the block surface 102-B is in contact with the floor surface, whereas face "AB" of the block surface 102-B does not touches the floor surface. In this position, the first sensor 108 may be adapted to sense a first parameter associated with the foot pedal 102. The first parameter may be sensed based upon an operative action (i.e. pressing action) performed by an operator or a technician or a physician, hereinafter referred to as a 'user'. In one exemplary embodiment, the fist parameter sensed may include the pressure applied, on the foot pedal 102. As illustrated in figure 1, a downward arrow 112 indicates the direction of pressing of the foot pedal 102, by the user, using a foot 114. In one embodiment, the foot pedal 102 may be pressed by the user in order to perform one or more medical operations. Upon sensing the first parameter, the first sensor 108 may transmit a first control signal to the control unit 104. The first control signal may be received in form of an analog signal indicating variation of the pressure applied at a plurality of predefined time intervals. Figure 4A illustrates the first control signal in form of the analog signal corresponding to the pressing action. As shown in figure 4A, the analog output indicates that the pressing varies according to the pressure exerted on the foot pedal 102.

After the receipt of the first control signal, the control unit 104 may be configured to perform a first function associated with one or more medical operations. In one embodiment, the first function performed by the control unit 104 may include generating a vacuum level, via an aspirator device (not shown in figures), based on the pressure applied on the foot pedal 102. The vacuum level may be generated in order to extract an oocyte from a patient's body during IVF treatment. Particularly, in the IVF treatment, the user may require a desired level of vacuum to be generated in order to extract oocytes from the patient's body. For this purpose, the user may press the foot pedal 102 thereby exerting pressure on the foot pedal 102. The pressure exerted by the user may be sensed by the first sensor 108. Upon sensing the pressure, the first sensor 108 may transmit the first control signal (e.g. a vacuum control signal) to the control unit 104. Upon receiving the first control signal from the first sensor 108, the control unit 104 may be enabled to control and/or vary various parameters in order to perform a specific function associated with the medical operation, i.e. controlling the level of the vacuum required for extracting the oocyte from the patient's body.

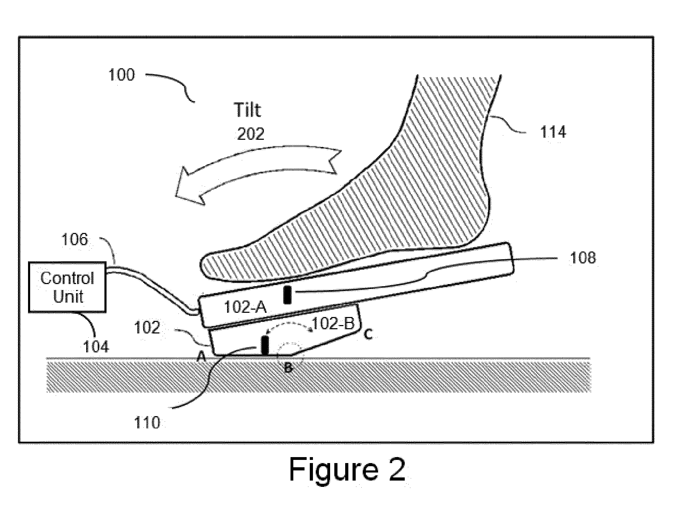

Now referring to figure 2, the foot pedal 102 is shown to be oriented in a second position (position-2). In one embodiment, in the postion-2, the plate 102-A along with the block surface 102-B of the foot pedal 102 may be positioned as shown in figure 2. As illustrated in figure 2, in the postion-2, the block surface 102-B may be tilted around the point 'B' on the block surface 102-B based upon an operative action (i.e. tilting action) performed by user. As shown in figure 2, the face 'AB' of the block surface 102-B is now in contact with the floor surface due to tilting of the block surface 102-B. In this position, the second sensor 110 may be adapted to sense a second parameter associated with the foot pedal 102. In one exemplary embodiment, the second parameter sensed may include the change in orientation of the foot pedal 102, by a predefined angle, from the first position to the second position. The foot pedal 102 may be tilted by the user using the foot 114. As illustrated in the figure 2, the arrow 202 indicates direction of the angular movement or the tilting of the block surface 102-B. In one embodiment, the foot pedal 102 may be tilted by the user in order to perform one or more medical operations. Upon sensing the second parameter, the second sensor 110 may transmit a second control signal to the control unit 104. The second control signal may be received in form of a digital signal indicating the orientation of the foot pedal 102 in the first position or the second position. Figure 4B illustrates the second control signal in form of the digital signal corresponding to the tilting action. As shown in figure 4B, the digital signal represents an output like that of an ON-OFF switch indicating the presence or absence of tilting action at a given point of time.

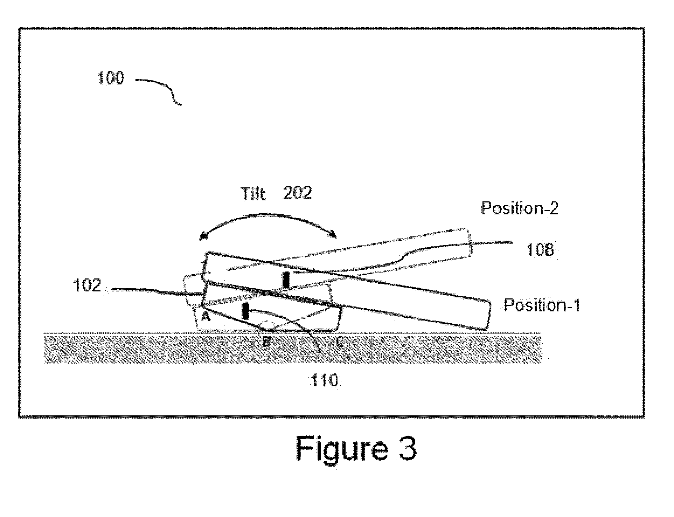

After the receipt of the second control signal, the control unit 104 may be configured to perform a second function associated with one or more medical operations. In one embodiment, the second function performed by the control unit 104 may include purging bore of a needle after the extraction of the oocyte from the patient's body. Figure 3 illustrates different positions (e.g. the position-1 and the position-2) of the foot switch 100 in accordance with various embodiments of the present disclosure. As shown in figure 3, the foot switch 100 may be interchangeably switched between the first position and the second position based upon the operative action performed by the user on the foot pedal 102. Such switching enables the control unit 104 to either perform the first function or the second function associated with the medical operation.

Although implementations of the electrical foot switch have been described in language specific to structural features and/or methods, it is to be understood that the appended claims are not necessarily limited to the specific features or methods described. Rather, the specific features and methods are disclosed as examples of implementations of the electrical foot switch.

## Claims

1. An electrical foot switch (100), comprising:
a foot pedal (102);
a first sensor (108) and a second sensor (110) positioned within the foot pedal (102), wherein the first sensor (108) and the second sensor (110) are adapted to sense a first parameter and a second parameter, respectively, associated with the foot pedal (102), wherein the first parameter and the second parameter are sensed when the foot pedal (102) is in a first position and a second position, respectively, and wherein the first parameter and the second parameter are sensed based upon an operative action performed by a user; and
a control unit (104) electrically coupled with the foot pedal (102), wherein the control unit (104) is configured to receive a first control signal or a second control signal from the first sensor (108) or the second sensor (110), respectively, wherein the first control signal is received when the foot pedal (102) is in the first position, and wherein the second control signal is received when the foot pedal (102) is in the second position, wherein the control unit (104) is further configured to perform a first function or a second function based upon the receipt of the first control signal or the second control signal respectively.

2. The electrical foot switch of claim 1, wherein the first sensor (108) is a pressure sensor selected from a group consisting of a strain-gauge sensor, a capacitive sensor, an electromagnetic sensor, a piezoelectric sensor, an optical sensor and a potentiometric sensor, and wherein the first parameter, sensed by the first sensor (108), is a pressure applied on the foot pedal (102) based upon the operative action performed by the user, and wherein the first control signal is an analog signal indicating variation of the pressure applied at a plurality of predefined time intervals.

3. The electrical foot switch of claim 1, wherein the second sensor (110) is a tilt sensor, and wherein the second parameter, sensed by the second sensor (110), is a change in orientation of the foot pedal (102), by a predefined angle, from the first position to the second position based upon the operative action performed by the user, and wherein the second control signal is a digital signal indicating the orientation of the foot pedal (102) in the first position or the second position.

4. The electrical foot switch of claim 2, wherein the first function comprises generating a vacuum level, via an aspirator device, based on the pressure applied on the foot pedal (102), and wherein the vacuum level is generated in order to extract an oocyte from a patient's body.

5. The electrical foot switch of claim 3, wherein the second function comprises purging bore of a needle after the extraction of the oocyte from the patient's body.

6. A method of operating an electrical foot switch, the method comprising:
sensing, via a first sensor (108) and a second sensor (110), a first parameter and a second parameter, respectively, associated with a foot pedal (102), wherein the first parameter and the second parameter are sensed when the foot pedal (102) is in a first position and a second position, respectively, and wherein the first parameter and the second parameter are sensed based upon an operative action performed by a user;
receiving, by a control unit (104), a first control signal or a second control signal from the first sensor (108) or the second sensor (110), respectively, wherein the first control signal is received when the foot pedal (102) is in the first position, and wherein the second control signal is received when the foot pedal (102) is in the second position; and
performing, by the control unit (104), a first function or a second function based upon the receipt of the first control signal or the second control signal respectively.

7. The method of claim 6, wherein the first sensor (108) is a pressure sensor selected from a group consisting of a strain-gauge sensor, a capacitive sensor, an electromagnetic sensor, a piezoelectric sensor, an optical sensor and a potentiometric sensor, and wherein the first parameter, sensed by the first sensor (108), is a pressure applied on the foot pedal (102) based upon the operative action performed by the user, and wherein the first control signal is an analog signal indicating variation of the pressure applied at a plurality of predefined time intervals.

8. The method of claim 6, wherein the second sensor (110) is a tilt sensor, and wherein the second parameter, sensed by the second sensor (110), is a change in orientation of the foot pedal (102), by a predefined angle, from the first position to the second position based upon the operative action performed by the user, and wherein the second control signal is a digital signal indicating the orientation of the foot pedal (102) in the first position or the second position.

9. The method of claim 7, wherein the first function comprises generating a vacuum level, via an aspirator device, based on the pressure applied on the foot pedal (102), wherein the vacuum level is generated in order to extract an oocyte from a patient's body.

10. The method of claim 8, wherein the second function comprises purging bore of a needle after the extraction of the oocyte from the patient's body.
